# EUROPEAN PATENT SPECIFICATION

(11) **EP 3 388 505 B1**
(45) Date of publication and mention of the grant of the patent: **01.04.2020**
(21) Application number: 15910253.2
(22) Date of filing: 10.12.2015
(51) Int. Cl.: C11D 1/14, A61K 8/46, A61Q 19/10, A61Q 5/02

(54) **SURFACTANT COMPOSITION**
TENSIDZUSAMMENSETZUNG
COMPOSITION COMPRENANT DES TENSIOACTIFS

(43) Date of publication of application: 17.10.2018
(73) Proprietor: Kao Corporation, Chuo-ku Tokyo 103-8210 (JP)
(72) Inventor: DOI, Yasuhiro, Wakayama-shi Wakayama 640-8580 (JP)
(74) Representative: Hoffmann Eitle
(86) International application number: PCT/JP2015/084686
(87) International publication number: WO 2017/098637

(56) References cited:
- GB-A- 2 123 882
- JP-A- S5 927 995
- JP-A- 2003 081 935
- JP-A- 2014 076 988
- JP-A- 2014 077 126
- JP-A- 2014 177 620
- JP-A- 2015 028 123
- JP-A- 2015 143 203
- US-A- 3 808 157
- US-A- 5 078 916
- US-A1- 2015 252 294
- US-B1- 6 184 190

## Description

### Field of the Invention.

The present invention relates to a surfactant composition comprising an internal olefin sulfonate in a high concentration.

### Background of the Invention

In general, the internal olefin sulfonate, a type of anionic surfactant, is obtained as follows: an internal olefin is allowed to react with sulfur trioxide gas to be sulfonated, the obtained sulfonic acid is neutralized, and then further hydrolyzed to yield the internal olefin sulfonate. Among others, an internal olefin sulfonate having 16 carbon atoms and an internal olefin sulfonate having 18 carbon atoms are known to be excellent in foamability and detergency, and have hitherto been used in various detergents.

On the other hand, such an internal olefin sulfonate is increased in viscosity with the increase of the concentration thereof, and accordingly may be degraded remarkably in handling. However, in order to improve freedom of the formulation design, handling and productivity in the final product, or in order to achieve the facilitation of the storage/transportation or the like of the final product, it is preferable to increase the concentration of the internal olefin sulfonate as much as possible while the manifestation of the desired effects is secured.

under such circumstances, for example, Patent Document 1 discloses a high concentration surfactant slurry comprising 55 to 75% by weight of an olefin sulfonic acid salt containing 50% by weight or more of an internal olefin sulfonate, 5 to 40% by weight of specific hydrocarbons such as olefins, and water; the achievement of the low viscosity of the obtained slurry has been attempted.

### Patent Document

(Patent Document 1) JP-A-sho 59-27995

US 2015/252294 describes a detergent composition comprising: at least one of a neat carboxylate, a neat sulfonate, or a neat sulfate heated to 30°C or greater, wherein the neat carboxylate (or acid thereof), the neat sulfonate (or acid thereof), or the neat sulfate (or acid thereof) is a solid or a viscous liquid at ambient temperature; a surfactant mixed with the neat carboxylate, neat sulfonate, or neat sulfate, wherein when the mixed composition is reduced to 5°X to 40°C. it is flowable; and one or more ingredients selected from at least one of one or more buffers, salts, etc.

US 3,808,157 discloses that olefin sulfonates obtained by saponifying the reaction product of SO₃ and olefins having from about 12 to about 16 carbon atoms per molecule and averaging about 14.1 to about 15.9 carbon atoms per molecule provide excellent hard water detergent materials when the olefins are predominantly unbranched 20 acyclic terminal monoolefins in admixture with from about 3 to about 30 mol percent of beta-branched terminal olefins and from about 3 to about 12 mol percent of internal olefins.

US 6,184,190 relates to a process for preparing an aqueous solution of a C14-C16 alpha olefin sulfonate, wherein the aqueous solution has a Klett color of less than 12, when diluted with water to a 5% solution.

GB 2123882 concerns a micellar slug for use in the recovery of oil, said slug consisting essentially of a hydrocarbon, an aqueous medium, a surfactant, and a cosurfactant, said surfactant containing, as an essential component, (a) at least one internal olefin sulfonate having 10 to 30 carbon atoms and (b) at least one alpha-olefin sulfonate having 10 to 30 carbon atoms.

### [Summary of the Invention]

The present invention relates to a surfactant composition comprising the following components (A), (B), and (C):
(A) 45.0% by mass or more and 70.0% by mass or less of an internal olefin sulfonate having 16 or more and 18 or less carbon atoms,
(B) 0.5% by mass or more and 25.0% by mass or less of an alpha-olefin sulfonate having 12 or more and 14 or less carbon atoms, and
(C) water,
wherein the total content of the component (A) and the component (B) is 66.0% by mass or more and 71.0% by mass or less.

When a final product such as a detergent is produced by using the high concentration surfactant slurry described in foregoing Patent Document 1, good foamability may not be secured due to the hydrocarbons such as olefins contained in the slurry. When an internal olefin sulfonate having 16 to 18 carbon atoms is adopted in order to enhance the performances such as foamability, in such a high concentration region as described in foregoing Patent Document 1, a phase separation, a solid deposition or the like occurs, accordingly the storage stability may be degraded, and the viscosity is increased to degrade the fluidity and to possibly make the handling of the slurry difficult. Therefore, there is still room for improvement in order to secure good storage stability and good fluidity while the concentration of the internal olefin sulfonate having 16 to 18 carbon atoms is increased.

Accordingly, the present inventor made various studies, and consequently found that a surfactant composition capable of securing a high fluidity and an excellent storage stability is obtained under the conditions that in a water-containing environment, while a high content of the internal olefin sulfonate having 16 or more and 18 or less carbon atoms is maintained, a specific amount of an alpha-olefin sulfonate having 12 or more and 14 or less carbon atoms is used in combination, and these ingredients are made to have a specific total content.

According to the surfactant composition of the present invention, the surfactant composition has a high fluidity and can exert excellent storage stability over a wide temperature region, while the internal olefin sulfonate having 16 or more and 18 or less carbon atoms capable of manifesting excellent foamability and an excellent detergency is contained in a high concentration. Therefore, the surfactant composition of the present invention is a surfactant composition highly useful and capable of being effectively used over wide fields as the base materials of final products for various detergents such as a fabric detergent, a dish detergent, a hair cleaning detergent, a body cleaning detergent, a precision component detergent, and a hard surface detergent.

### [Detailed Description of the Invention]

Hereinafter, the present invention is described in detail.

The surfactant composition of the present invention comprises the following components (A), (B), and (C):
(A) 45.0% by mass or more and 70.0% by mass or less of an internal olefin sulfonate having 16 or more and 18 or less carbon atoms,
(B) 0.5% by mass or more and 25.0% by mass or less of an alpha-olefin sulfonate having 12 or more and 14 or less carbon atoms, and
(C) water,
in which the total content of the component. (A) and the component (B) is 66.0% by mass or more and 71.0% by mass or less.

The reason for the fact that the surfactant composition of the present invention manifests good fluidity and good storage stability is uncertain, but it may be considered as follows.

In general, in the composition comprising a high concentration of the internal olefin sulfonate, having 16 or more and 18 or less carbon atoms, of the component (A), two phases, namely a micelle phase and a lamellar phase are present in the composition system, and the phase separation is considered to occur due to the specific gravity difference between these phases; however, in the present invention, for the component (B), an alpha-olefin sulfonate having 12 or more and 14 or less carbon atoms, shorter in the carbon chain is included in a specific amount, consequently such a micelle phase is reduced or vanished, and thus the phase separation is considered to be effectively suppressed. On the other hand, although the alpha-olefin sulfonates having 16 or more and 18 or less carbon atoms included in the internal olefin sulfonate having 16 or more and 18 or less carbon atoms in the component (A) may form a deposit, because the solubility is improved due to the alpha-olefin sulfonates having 12 or more and 14 or less carbon atoms in the component (B) shorter in carbon chain, and thus the production of the deposit is considered to be effectively suppressed. Moreover, the micelle phase is reduced or vanished, a mixed lamella phase including such a component (B) is formed, and thus the fluidity is also considered to be improved.

### <Internal Olefin Sulfonate (A)>

The surfactant composition of the present invention comprises an internal olefin sulfonate having 16 or more and 18 or less carbon atoms (hereinafter, also referred to as the component (A)) in a content of 45.0% by mass or more and 70.0% by mass or less, from the viewpoint of being excellent in the foamability and detergency in the final products such as detergents using such a composition.

In the present invention, the internal olefin sulfonate is a sulfonate obtained by sulfonation, neutralization and hydrolysis of a raw material, namely, an internal olefin (an olefin having a double bond in the interior of the olefin chain) having 16 or more and 18 or less carbon atoms. Such an internal olefin includes a case where a so-called alpha-olefin having the position of the double bond at the position 1 of the carbon chain (hereinafter, also referred to as α-olefin) is included in a small amount. In addition, when an internal olefin is sulfonated, β-sultone is produced qualitatively, a fraction of the β-sultone is converted into γ-sultone, and an olefin sulfonic acid, and further these are converted, in the neutralization/hydrolysis step, into a hydroxy alkane sulfonate and an olefin sulfonate (for example, J. Am. Oil Chem. Soc. 69, 39(1992)). Here, the hydroxy group of the obtained hydroxy alkane sulfonate is located in the interior of the alkane chain, and the double bond of the olefin sulfonate is located in the interior of the olefin chain. In addition, the obtained product is mainly a mixture composed of these, and in a fraction of the product, a hydroxy alkane sulfonate having a hydroxy group at the terminal of the carbon chain, or an olefin sulfonate having the double bond at a terminal of the carbon chain is included in a small amount as the case may be. In the present description, these respective products and the mixture of these products are collectively referred to as the internal olefin sulfonate (component (A)). In addition, the hydroxy alkane sulfonate is referred to as the hydroxy form of the internal olefin sulfonate (hereinafter, also referred to as HAS), and the olefin sulfonate is referred to as the olefin form of the internal olefin sulfonate (hereinafter, also referred to as IOS).

The number of the carbon atoms of the internal olefin sulfonate of the component (A) is 16 or more from the viewpoint of having good foamability, good detergency and good rinsability. In addition, the number of the carbon atoms of the internal olefin sulfonate of the component (A) is 18 or less from the viewpoint of manifesting fluidity at high concentration. The number of the carbon atoms of the internal olefin sulfonate of the component (A) is preferably 16 or 18 from the viewpoint of the availability.

When the internal olefin sulfonate having 16 carbon atoms and the internal olefin sulfonate having 18 carbon atoms of the component (A) are used as mixed with each other, the mass ratio of the content of the internal olefin sulfonate having 16 carbon atoms to the content of the internal olefin sulfonate having 18 carbon atoms, (internal olefin sulfonate having 16 carbon atoms/internal olefin sulfonate having 18 carbon atoms), is preferably from 100/0 to 40/60, more preferably from 95/5 to 50/50, and further preferably from 90/10 to 70/30, from the viewpoint of having good foamability, good detergency and good rinsability.

The mass ratio can be measured by using a high performance liquid chromatography-mass spectrometer (hereinafter, abbreviated as HPLC-MS). Specifically, the internal olefin sulfonate having 16 carbon atoms and the internal olefin sulfonate having 18 carbon atoms are separated from the component (A) or the obtained surfactant composition by HPLC, the separated internal olefin sulfonates are identified by MS, and the mass ratio can be determined from the HPLC-MS peak areas of the internal olefin sulfonates.

Examples of the internal olefin sulfonate include an alkali metal salt, an alkaline earth metal (1/2 atom) salt, and an ammonium salt or an organic ammonium salt. Examples of the alkali metal salt include an Na salt and a K salt.

As can be seen from the above-described production method, the sulfonate group of the internal olefin sulfonate of the component (A) is located in the interior of the carbon chain of the internal olefin sulfonate, namely, the olefin chain or the alkane chain, and a fraction of the internal olefin sulfonate of the component (A) includes a small amount of olefin sulfonates each having a sulfonate group at a terminal of the carbon chain as the case may be. In the present invention, from the viewpoint of the storage stability, it is preferable to contain a low amount of the internal olefin sulfonate locating the position of such a sulfonate group at the position 2 of the carbon chain, and to contain a high amount of the internal olefin sulfonates locating such a sulfonate group at a more internal position.

The content of the internal olefin sulfonate having the sulfonate group at the position 2 in the component (A) is preferably 30.0% by mass or less, more preferably 28.0% by mass or less, and further preferably 25.0% by mass or less, in the component (A), from the viewpoint of storage stability. In addition, the content of the internal olefin sulfonate having the sulfonate group at the position 2 in the component (A) is preferably 10.0% by mass or more, more preferably 15.0% by mass or more, and further preferably 20.0% by mass or more, in the component (A), from the viewpoint of improving productivity. Additionally, the content of the internal olefin sulfonate having the sulfonate group at the position 2 in the component (A) is preferably 10.0% by mass or more and 30.0% by mass or less, more preferably 15.0% by mass or more and 28.0% by mass or less, and further preferably 20.0% by mass or more and 25.0% by mass or less, in the component (A), from the viewpoint of storage stability, the viewpoint of having a viscosity minimum point manifesting fluidity at a high concentration, and the viewpoint of improving productivity.

The content of the olefin sulfonate having the sulfonate group at the position 1 of the olefin chain or the alkane chain in the component (A) is preferably 3.0% by mass or less, more preferably 2.5% by mass or less, and further preferably 2.0% by mass or less, in the component (A), from the viewpoint of storage stability, and is preferably 0.01% by mass or more, in the component (A), from the viewpoint of reducing production cost and improving productivity.

The content of the internal olefin sulfonate having the sulfonate group at the position 3 or a more inner position of the olefin chain or the alkane chain in the component (A) is preferably 60.0% by mass or more, more preferably 65.0% by mass or more, and further preferably 70.0% by mass or more, in the component (A), from the viewpoint of storage stability.

The internal olefin sulfonate is preferably a mixture composed of the hydroxy form and the olefin form, from the viewpoint of improving productivity and reducing impurities. The mass ratio of the content of the hydroxy form of the internal olefin sulfonate to the content of the olefin form of the internal olefin sulfonate, (hydroxy form/olefin form), in the component (A) or the surfactant composition is preferably from 50/50 to 100/0, more preferably from 60/40 to 100/0, further preferably from 70/30 to 100/0, and further preferably from 75/25 to 100/0, and more preferably from 75/25 to 95/5, from the viewpoint of improving productivity and reducing impurities.

The mass ratio of the content of the hydroxy form of the internal olefin sulfonate to the content of the olefin form of the internal olefin sulfonate in the component (A) can be measured by the method described in Example after separating the hydroxy form and the olefin form by HPLC from the component (A) or the surfactant composition to be obtained.

The content of the component (A) is 45.0% by mass or more, preferably 61.0% by mass or more, more preferably 62.0% by mass or more, further preferably 63.0% by mass or more, and furthermore preferably 64.0% by mass or more, in the surfactant composition of the present invention, from the viewpoint of storage stability and the viewpoint of enhancing freedom of the formulation design in the final product using the surfactant composition of the present invention. The content of the component (A) is 70.0% by mass or less, preferably 69.0% by mass or less, more preferably 68.0% by mass or less, further preferably 67.0% by mass or less, and furthermore preferably 66.0% by mass or less, in the surfactant composition of the present invention, from the viewpoint of fluidity. In addition, the content of the component (A) is 45.0% by mass or more and 70.0% by mass or less in the surfactant composition of the present invention; the content of the component (A) is preferably from 61.0 to 70.0% by mass, more preferably from 62.0 to 70.0% by mass, further preferably from 62.0 to 69.0% by mass, furthermore preferably from 63.0 to 68.0% by mass, furthermore preferably from 64.0 to 67.0% by mass, and furthermore preferably from 64.0 to 66.0% by mass, in the surfactant composition of the present invention, further from the viewpoint of exerting, in a well-balanced manner, all of the storage stability, the improvement in freedom of the formulation design in the final product using the surfactant composition of the present invention, and the fluidity.

The content of the component (A) is also further preferably 64.0% by mass or more, furthermore preferably 66.0% by mass or more, furthermore preferably 67.0% by mass or more, furthermore preferably 68.0% by mass or more, and 70.0% by mass or less, in the surfactant composition of the present invention, mainly from the viewpoint of storage stability, and the viewpoint of enhancing freedom of the formulation design in the final product using the surfactant composition of the present invention. In addition, the content of the component (A) is further preferably from 64.0 to 70.0% by mass, furthermore preferably from 66.0 to 70.0% by mass, furthermore preferably from 67.0 to 70.0% by mass, and furthermore preferably from 68.0 to 70.0% by mass, in the surfactant composition of the present invention, mainly from the viewpoint of storage stability, and the viewpoint of enhancing freedom of the formulation design in the final product using the surfactant composition of the present invention.

The content of the component (A) is further preferably 62.0% by mass or more, further preferably 68.0% by mass or less, furthermore preferably 67.0% by mass or less, furthermore preferably 66.0% by mass or less, and furthermore preferably 65.0% by mass or less, in the surfactant composition of the present invention, mainly from the viewpoint of fluidity. In addition, the content of the component (A) is further preferably from 62.0 to 68.0% by mass, furthermore preferably from 62.0 to 67.0% by mass, furthermore preferably from 62.0 to 66.0% by mass, and furthermore preferably from 62.0 to 65.0% by mass, in the surfactant composition of the present invention, mainly from the viewpoint of fluidity.

The component (A) can be produced by sulfonation, neutralization and hydrolysis of a raw material, namely, an internal olefin having 16 or more and 18 or less carbon atoms.

The sulfonation reaction can be performed by allowing from 1.0 to 1.2 moles of sulfur trioxide gas to react with 1 mole of the internal olefin. The sulfonation reaction can be performed at a reaction temperature of from 20 to 40°C.

The neutralization is performed by allowing an alkaline aqueous solution containing an alkaline substance such as sodium hydroxide, ammonia, or 2-aminoethanol in an amount of from 1.0 to 1.5 times as much as the theoretical value of moles of the sulfonate group. The hydrolysis reaction may be performed in the presence of water at 90 to 200°C for from 30 minutes to 3 hours. These reactions can be performed continuously. In addition, after the completion of the reactions, purification can be performed, for example, by extraction and washing.

In the production of the internal olefin sulfonate(A), a raw material internal olefin having a distribution over the number of the carbon atoms of 16 to 18 may be used for performing the sulfonation, neutralization and hydrolysis treatments, a raw material internal olefin having a single number of carbon atoms may be used for performing the sulfonation, neutralization and hydrolysis treatments, or alternatively, if necessary, multiple types of preliminarily produced internal olefin sulfonates different from each other in the number of carbon atoms may also be mixed with each other.

As described above, the internal olefin as referred to in the present invention means an olefin having a double bond in the interior of the olefin chain. The number of the carbon atoms in the internal olefin is 16 or more and 18 or less. The internal olefins to be used may be used each alone or in combinations of two or more thereof.

When the internal olefin sulfonate of the component (A) is obtained by sulfonating, neutralizing and hydrolyzing a raw material internal olefin, the total content of the internal olefins having a double bond at the position 2 in the raw material internal olefin is preferably 45.0% by mass or less, more preferably 40.0% by mass or less, and further preferably 35.0% by mass or less, in the raw material internal olefin, from the viewpoint of storage stability and fluidity. The total content of the internal olefins having a double bond at the position 2 in the raw material internal olefin is preferably 10.0% by mass or more, more preferably 15.0% by mass or more, further preferably 20.0% by mass or more, and further preferably 25.0% by mass or more, in the raw material internal olefin, from the viewpoint of reducing production cost and improving productivity. In addition, the total content of the internal olefins having a double bond at the position 2 in the raw material internal olefin is preferably 10.0% by mass or more and 45.0% by mass or less, more preferably 15.0% by mass or more and 40.0% by mass or less, further preferably 20.0% by mass or more and 35.0% by mass or less, and further preferably 25.0% by mass or more and less than 35.0% by mass, in the raw material internal olefin, from the viewpoint of storage stability, fluidity and improving productivity.

The content of the olefin having a double bond at the position 1, namely a so-called alpha-olefin in the raw material internal olefin is preferably 3.0% by mass or less, more preferably 2.8% by mass or less, further preferably 2.5% by mass or less, further preferably 2.0% by mass or less, further preferably 1.5% by mass or less, and furthermore preferably 1.0% by mass or less, in the raw material internal olefin, from the viewpoint of storage stability and fluidity, and is preferably 0.01% by mass or more, in the raw material internal olefin, from the viewpoint of reducing production cost and improving productivity.

The total content of the internal olefins having a double bond at the position 3 or a more inner position in the raw material internal olefin is preferably 50.0% by mass or more, more preferably 60.0% by mass or more, and further preferably 65.0% by mass or more, in the raw material internal olefin, from the viewpoint of imparting storage stability and fluidity.

The distribution of the double bond in the raw material internal olefin can be measured by using, for example, a gas chromatograph-mass spectrometer (hereinafter, abbreviated as GC-MS). Specifically, the components different from each other in the carbon chain length and the double bond position are accurately separated by using a gas chromatograph analyzer (hereinafter, abbreviated as GC), each of the separated components is analyzed by using a mass analyzer (hereinafter, abbreviated as MS), to allow the position of the double bond to be identified, and the GC peak areas allow the proportions of the components to be determined.

### <Alpha-Olefin Sulfonate (B)>

The surfactant composition of the present invention includes, from the viewpoint of storage stability and fluidity, 0.5% by mass or more and 25.0% by mass or less of an alpha-olefin sulfonate (hereinafter, also referred to as the component (B)) having 12 or more and 14 or less carbon atoms. The number of the carbon atoms in the alpha-olefin sulfonate of the component (B) is preferably from 12 or 14 from the viewpoint of availability, and is preferably 14 from the viewpoint of storage stability and fluidity.

Examples of the salt of the alpha-olefin sulfonate include an alkali metal salt, an alkaline earth metal (1/2 atom) salt, an ammonium salt or an organic ammonium salt. Examples of the alkali metal salt include a Na and a K salt.

The component (B) can be produced by sulfonation, neutralization and hydrolysis of a raw material, namely, an alpha-olefin (an olefin having the double bond at a terminal of the carbon chain) having 12 or more and 14 or less carbon atoms. The alpha-olefin which is the raw material of the component (B) may be a simple substance having 12 or 14 carbon atoms, a mixture of these, or a mixture of alpha-olefins having other numbers of carbon atoms. Specific examples of the commercially available products of such alpha-olefins include: "LIPOLAN LB-440", "LIPOLAN PJ-400", and "LIPOLAN PB-800", manufactured by Lion Corporation"; "ALFANOX 46" manufactured by Kao Corporation; and "Hostarpur OSB" manufactured by Clariant GmbH.

The content of the component (B) is 0.5% by mass or more, preferably 1.0% by mass or more, more preferably 2.0% by mass or more, further preferably 3.0% by mass or more, and furthermore preferably 4.0% by mass or more, in the surfactant composition of the present invention, from the viewpoint of storage stability. In addition, the content of the component (B) is 25.0% by mass or less, preferably 9.0% by mass or less, more preferably 8.0% by mass or less, further preferably 7.0% by mass or less, and furthermore preferably 6.0% by mass or less, in the surfactant composition of the present invention, from the viewpoint of fluidity and the viewpoint of enhancing freedom of the formulation design in the final product using the surfactant composition of the present invention. In addition, the content of the component (B) is 0.5% by mass or more 25.0% by mass or less, preferably from 1.0 to 9.0% by mass, more preferably from 1.0 to 8.0% by mass, further preferably from 2.0 to 7.0% by mass, furthermore preferably from 3.0 to 6.0% by mass, and furthermore preferably from 4.0 to 6.0% by mass, in the surfactant composition of the present invention.

The total content of the component (A) and the component (B), ((A)+(B)), is 66.0% by mass or more, preferably 68.0% by mass or more, more preferably 68.5% by mass or more, and further preferably 69.0% by mass or more, in the surfactant composition of the present invention, from the viewpoint of the storage stability of the surfactant composition of the present invention. The total content of the component (A) and the component (B), ((A)+(B)), is 71.0% by mass or less, and preferably 70.0% by mass or less, in the surfactant composition of the present invention, from the viewpoint of fluidity. In addition, the total content of the component (A) and the component (B), ((A)+(B)), is 66.0% by mass or more and 71.0% by mass or less, preferably from 68.0 to 70.0% by mass, more preferably from 68.5 to 70.0% by mass, and further preferably from 69.0 to 70.0% by mass, in the surfactant composition of the present invention, from the viewpoint of exerting, in a well-balanced manner, the storage stability and the fluidity of the surfactant composition of the present invention.

The total content of the component (A) and the component (B), ((A)+(B)), is 66.0% by mass or more, preferably 69.5% by mass or less, more preferably 69.0% by mass or less, and further preferably 68.5% by mass or less, in the surfactant composition of the present invention, mainly from the viewpoint of fluidity. In addition, the total content of the component (A) and the component (B), ((A)+(B)), is preferably from 66.0 to 69.5% by mass, more preferably from 66.0 to 69.0% by mass, and further preferably from 66.0 to 68.5% by mass, in the surfactant composition of the present invention, mainly from the viewpoint of fluidity.

The mass ratio of the content of the component (A) to the content of the component (B), ((A)/(B)), is preferably 1.8 or more, more preferably 2.5 or more, further preferably 5.0 or more, furthermore preferably 10.0 or more, and furthermore preferably 12.5 or more, from the viewpoint of enhancing freedom of the formulation design in the final product using the surfactant composition of the present invention. The mass ratio of the content of the component (A) to the content of the component (B), ((A)/(B)), is preferably 140 or less, more preferably 75.0 or less, further preferably 40.0 or less, furthermore preferably 25.0 or less, furthermore preferably 18.0 or less, and furthermore preferably 15.0 or less, from the viewpoint of fluidity. In addition, the mass ratio of the content of the component (A) to the content of the component (B), ((A)/(B)), is preferably from 1.8 to 140, more preferably from 2.5 to 75.0, further preferably from 5.0 to 40.0, furthermore preferably from 5.0 to 25.0, furthermore preferably from 10.0 to 18.0, and furthermore preferably from 12.5 to 15.0, from the viewpoint of exerting, in a well-balanced manner, the improvement of freedom of the formulation design in the final product using the surfactant composition of the present invention, and fluidity.

The mass ratio of the content of the component (A) to the content of the component (B), ((A)/(B)), is preferably 10.0 or more, more preferably 20.0 or more, further preferably 40.0 or more, and preferably 75.0 or less, mainly from the viewpoint of enhancing freedom of the formulation design in the final product using the surfactant composition of the present invention. In addition, the mass ratio of the content of the component (A) to the content of the component (B), ((A)/(B)), is preferably from 10.0 to 75.0, more preferably from 20.0 to 75.0, and further preferably from 40.0 to 75.0, mainly from the viewpoint of enhancing freedom of the formulation design in the final product using the surfactant composition of the present invention.

The surfactant composition of the present invention comprises water (hereinafter, also referred to as the component (C)). Such water is not particularly limited, but is preferably purified water such as ion-exchanged water, distilled water, reverse osmosis membrane water, and pure water.

The content of the component (C) is preferably 34.0% by mass or less, more preferably 32.0% by mass or less, further preferably 31.0% by mass or less, and furthermore preferably 30.5% by mass or less, in the surfactant composition of the present invention, from the viewpoint of the storage stability of the surfactant composition of the present invention. The content of the component (C) is preferably 24.0% by mass or more, more preferably 25.0% by mass or more, and furthermore preferably 26.0% by mass or more, in the surfactant composition of the present invention, from the viewpoint of fluidity. In addition, the content of the component (C) is preferably from 24.0 to 34.0% by mass, more preferably from 25.0 to 32.0% by mass, furthermore preferably from 25.0 to 31.0% by mass, and furthermore preferably from 26.0 to 30.5% by mass, in the surfactant composition of the present invention, from the viewpoint of storage stability and fluidity.

### <Other Components>

As described above, the component (A) is obtained by sulfonating, neutralizing and hydrolyzing an internal olefin, and accordingly there is a possibility that the unreacted raw material internal olefin and unreacted inorganic compounds remain in the surfactant composition of the present invention. It is preferable to contain a lower amount of these components.

The content of the unreacted raw material internal olefin in the surfactant composition of the present invention is preferably 5.0% by mass or less, more preferably 3.0% by mass or less, further preferably 2.0% by mass or less, and furthermore preferably 1.5% by mass or less, in the surfactant composition of the present invention, from the viewpoint of the foamability, the detergency and the like in the final product using the surfactant composition of the present invention.

The content of the unreacted raw material internal olefin can be measured by the methods described in Examples.

The content of the inorganic compounds in the surfactant composition of the present invention is preferably 5.0% by mass or less, more preferably 3.0% by mass or less, further preferably 2.0% by mass or less, and furthermore preferably 1.5% by mass or less, in the surfactant composition of the present invention, from the viewpoint of the foamability, the detergency and the like in the final product using the surfactant composition of the present invention.

Here, the inorganic compounds include a sulfate, and an alkaline agent, and the contents of these inorganic compounds can be measured by potentiometric titration. Specifically, the contents of these inorganic compounds can be measured by the method described in Examples.

The surfactant composition of the present invention may include a surfactant (hereinafter, also referred to as the component (D)) other than the component (A) and the component (B), within a range not impairing the advantageous effects of the present invention. For such a surfactant of the component (D), it is possible to use any surfactant conventionally used in medicines, quasi-medicines, cosmetics, toiletries, miscellaneous goods and the like, and other than the component (A) and the component (B); specific examples of such a surfactant include an anionic surfactant, a nonionic surfactant, an amphoteric surfactant, and a cationic surfactant. Among such components (D), the surfactant of the component (D) is preferably an anionic surfactant, a nonionic surfactant or an amphoteric surfactant, from the viewpoint of detergency, foamability and foam quality improvement.

The surfactant composition of the present invention may appropriately include, in addition to the above-described components, a viscosity reducing agent, a polyhydric alcohol, a preservative agent, and a reducing agent, and additionally, other components conventionally used as raw materials for final products such as detergents. Examples of such components include a feeling improver, a thickener, a fragrance, an ultraviolet absorber, a visible light absorber, a chelating agent, an antioxidant, a colorant, a preservative, a pH adjuster, a viscosity adjuster, a pearl gloss agent, and a humectant.

From the viewpoint of having good fluidity and excellent storage stability in combination, and the viewpoint of enhancing freedom of the formulation design in the final product using the surfactant composition of the present invention, the viscosity at 25°C of the surfactant composition of the present invention is preferably 5 Pa·s or more, more preferably 10 Pa·s or more, further preferably 30 Pa·s or more, and furthermore preferably 60 Pa·s or more, and furthermore preferably 68 Pa·s or more, and preferably 155 Pa·s or less, more preferably 130 Pa·s or less, further preferably 110 Pa·s or less, furthermore preferably 95 Pa·s or less, and furthermore preferably 80 Pa·s or less. In addition, the viscosity at 25°C of the surfactant composition of the present invention is preferably from 5 to 155 Pa·s, more preferably from 10 to 130 Pa·s, further preferably from 30 to 110 Pa·s, furthermore preferably from 60 to 95 Pa·s, and furthermore preferably from 68 to 80 Pa·s.

The viscosity at 25°C of the surfactant composition of the present invention means the value measured by the method described in Examples.

### <Method for Producing Surfactant Composition>

The production method of the surfactant composition of the present invention is not particularly limited, and the surfactant composition of the present invention can be produced by a conventional method. Specifically, for example, the component (A), the component (B), if necessary the component (D) or other components, and water (component (C)) are heated and uniformly mixed with each other. The component (A) may be, if necessary, preliminarily dispersed or dissolved in water and then added.

### <Applications, and Method of Application>

The surfactant composition of the present invention can be suitably used as a base material for various detergents which are excellent in stability and fluidity. Specific examples of such a detergent include a fabric detergent, a dish detergent, a hair cleaning detergent, a body cleaning detergent, a precision component detergent, a hard surface detergent, and the like. These detergents can be produced by mixing the surfactant composition of the present invention, and if necessary, other components together with water.

The other components mean a surfactant other than the component (A) and the component (B), and the components conventionally used as the raw materials for the various detergents which are final products.

### [Examples]

Hereinafter, the present invention is specifically described on the basis of Examples. The contents of the individual components are given in percent by mass, unless otherwise specified in the tables.

The measurements methods of the physical properties are as follows.

### (1) Measurement conditions

### (i) Measurement method of the position of the double bond in the raw material internal olefin

The position of the double bond in the internal olefin was measured by gas chromatography (hereinafter, abbreviated as GC). Specifically, dimethyl disulfide was allowed to react with an internal olefin to obtain a dithio derivative of the internal olefin, and then the individual components were separated by GC. Consequently, the position of the double bond was determined from the peak areas of the respective components.

The apparatus used for the measurement and the analysis conditions are as follows. GC apparatus: "HP6890" (manufactured by Hewlett-Packard Company), Column: "Ultra-Alloy-1HT capillary column" (30 m × 250 µm × 0.15 µm, manufactured by Frontier Laboratories Ltd.), Detector: (hydrogen flame ion detector (FID)), Injection temperature: 300°C, Detector temperature: 350°C, He flow rate: 4.6 mL/min

### (ii) Measurement method of mass ratio of hydroxy form/olefin form

The mass ratio of hydroxy form/olefin form was measured by HPLC-MS. Specifically, the hydroxy form and the olefin form were separated by HPLC, and each of the hydroxy form and the olefin form was identified by using a MS. Consequently, the proportions of the respective forms were determined from the HPLC-MS peak areas of the respective forms.

The apparatus and the conditions used for the measurement are as follows. HPLC apparatus: "Agilent Technologies 1100" (manufactured by Agilent Technologies, Inc.), Column: "L-column ODS" (4.6 × 150 mm, manufactured by General Foundation of Chemicals Evaluation and Research Institute), Sample preparation (dilution with methanol by a factor of 1,000), Eluent A (10 mM ammonium acetate-added water), Eluent B (10 mM ammonium acetate-added methanol), Gradient (0 min (A/B=30/70%)→10 min (30/70%)→55 min (0/100%)→65 min (0/100%)→66 min (30/70%)→75 min (30/70%), MS apparatus "Agilent Technologies 1100MS SL (G1946D)" (manufactured by Agilent Technologies, Inc.), MS detection: (Anion detection, m/z60-1600, UV 240 nm)

### (iii) Measurement method of the content of the raw material internal olefin

The content of the raw material internal olefin was measured by GC. Specifically, ethanol and petroleum ether were added to an aqueous solution of the internal olefin sulfonate, and then the olefin was obtained in the petroleum ether phase by extraction. Consequently, the amount of the raw material internal olefin was quantitatively determined from the GC peak area of the olefin. The apparatus and the analysis conditions used in the measurement are as follows. GC apparatus: "Agilent Technologies 6850" (manufactured by Agilent Technologies, Inc.), Column: "Ultra-Alloy-1HT capillary column" (15 m × 250 µm × 0.15 µm, manufactured by Frontier Laboratories Ltd.), Detector: (hydrogen flame ion detector (FID)), Injection temperature: 300°C, Detector temperature: 350°C, He flow rate: 3.8 mL/min

### (iv) Measurement method of the contents of inorganic compounds

The contents of the inorganic compounds were measured by potentiometric titration or neutralization titration. Specifically, the content of Na₂SO₄ was quantitatively determined by determining the content of the sulfate radical (SO₄²⁻) by the potentiometric titration using a SO₄ electrode. The content of NaOH was quantitatively determined by neutralization titration using a diluted hydrochloric acid.

### (2) Production of internal olefin

### [Production Example A]

In a flask equipped with a stirring device, 15,000 g (61.9 moles) of 1-hexadecanol "KALCOL 6098" (manufactured by Kao Corporation), and 750 g (5% by mass in relation to the raw material alcohol) of γ-alumina (Strem Chemical, Inc.) as a solid acid catalyst were placed, and were allowed to react under stirring at 280°C for 8 hours while nitrogen (7,000 mL/min) was being allowed to flow in the reaction system. The alcohol conversion rate after the completion of the reaction was 100%, and the purity of the C16 internal olefin was 99.7%. The obtained crude internal olefin was transferred into a distillation flask, and distilled at 136 to 160°C/4.0 mmHg to yield an internal olefin having 16 carbon atoms in a purity of 100%. The distribution of the double bond in the obtained internal olefin was 1.6% by mass at the C1 position, 29.7% by mass at the C2 position, 24.9% by mass at the C3 position, 18.7% by mass at the C4 position, 12.2% by mass at the C5 position, 6.7% by mass at the C6 position, and 6.2% by mass as the total proportion at the C7 and C8 positions.

### [Production Example B]

In a flask equipped with a stirring device, 15,000 g (55.5 moles) of 1-octadecanol "KALCOL 8098" (manufactured by Kao Corporation), and 750g (5% by mass in relation to the raw material alcohol) of γ-alumina (Strem Chemicals, Inc.) as a solid acid catalyst were placed, and were allowed to react under stirring at 280°C for 12 hours while nitrogen (7,000 mL/min) was being allowed to flow in the reaction system. The alcohol conversion rate after the completion of the reaction was 100%, and the purity of the C18 internal olefin was 98.5%. The obtained crude internal olefin was transferred into a distillation flask, and distilled at 148 to 158°C/0.5 mmHg to yield an internal olefin having 18 carbon atoms in a purity of 100%. The distribution of the double bond in the obtained internal olefin was 1.6% by mass at the C1 position, 30.6% by mass at the C2 position, 24.8% by mass at the C3 position, 18.6% by mass at the C4 position, 11.1% by mass at the C5 position, 6.5% by mass at the C6 position, 3.7% at the C7 position, and 3.0% by mass as the total proportion at the C8 and C9 positions.

### (3) Production of internal olefin sulfonates

### [Production Example 1]

The internal olefins having 16 and 18 carbon atoms respectively prepared in Production Examples A and B were mixed with each other so as for the mass ratio to be 8:2, and the sulfonation reaction was performed by allowing the obtained internal olefin to react with a sulfur trioxide gas having a SO₃ concentration of 2.8% by volume by using a thin film-type sulfonation reactor (inner diameter: 14 mmφ, length: 4 m), and by allowing cold water at 10°C to pass the outer jacket of the reactor. The reaction molar ratio SO₃/internal olefin was set to be 1.02.

The obtained sulfonated product was added to an alkaline aqueous solution to which sodium hydroxide was added in an amount of 1.2 times the theoretical acid value in terms of molar number, and the sulfonated product was neutralized at 30°C for 1 hour under stirring. The neutralized product was hydrolyzed by heating at 160°C for 1 hour in an autoclave, then the moisture was removed by freeze-drying, and thus a sodium internal olefin sulfonate composition (IOS-1) having a carbon number ratio C16/C18 = 8/2 (mass ratio) was obtained. In this IOS-1, the content of the component (A) was 96.7% by mass, the content of the raw material internal olefin was 1.8% by mass, and the content of the inorganic compounds was 1.5% by mass. The mass ratio of hydroxy form/olefin form in the component (A) was 85/15, the content of the alpha-olefin sulfonate in the component (A) was 1.9% by mass, and the content of the internal olefin sulfonate having a hydrophilic group at the position 2 in the component (A) was 23.0% by mass.

### [Production Example 2]

A sodium internal olefin sulfonate composition (IOS-2) having 16 carbon atoms was obtained under the same conditions as in Production Example 1, except that the internal olefin having 16 carbon atoms produced in Production Example A. In the IOS-2, the content of the component (A) was 97.2% by mass, the content of the raw material internal olefin was 1.5% by mass, and the content of the inorganic compounds was 1.3% by mass. The mass ratio of hydroxy form/olefin form in the component (A) was 85/15, the content of the alpha-olefin sulfonate in the component (A) was 2.0% by mass, and the content of the internal olefin sulfonate having a hydrophilic group at the position 2 in the component (A) was 22.0% by mass.

### (4) Production of alpha-olefin sulfonate

### [Production Example 3]

An alpha-olefin sulfonate having 14 carbon atoms "LIPOLAN LB-440" (effective component: 36%, manufactured by Lion Corporation) was freeze-dried to remove the moisture, and thus AOS-1 was obtained. In AOS-1, the content of the moisture was water was 0% by mass, and the content of the sodium alpha-olefin sulfonate having 14 carbon atoms was 92.5% by mass.

### [Production Example 4]

A sodium alpha-olefin sulfonate "ALFANOX 46" (content of the component having 14 carbon atoms: 60% by mass, effective component: 38%, manufactured by Kao Corporation) having 14 and 16 carbon atoms was freeze-dried to remove the moisture, and thus AOS-2 was obtained. In AOS-2, the moisture was 0% by mass, and the content of the sodium alpha-olefin sulfonate having 14 and 16 carbon atoms was 94.8% by mass.

### [Examples 1 to 13 and Comparative Examples 1 to 9]

According to the compositions shown in Table 1, the internal olefin sulfonates (the components (A)) obtained by Production Examples 1 and 2, an alpha-olefin sulfonate (the component (B)), and purified water were used, and these were mixed uniformly at 80°C, placed in sealed containers, and were allowed to stand still over one night in a thermostatic bath at 80°C. Subsequently, the obtained products were stirred at 50°C or higher, and each of the obtained surfactant compositions was placed in three 50-mL centrifuge tubes in an amount of 20 g in each tube.

The centrifuge tubes were stored at 40°C, 25°C, and 15°C respectively for 1 day, and the fluidity was evaluated according to the following method, and the viscosity was also measured and evaluation of the storage stability was also performed.

Table 1 shows the compositions and evaluation results of the measured surfactant compositions.

### <<Evaluation of Fluidity>>

The surfactant composition stored at 25°C was subjected to centrifugal defoaming by using the "Hitachi high speed refrigerated centrifuge CR21GIII" (Rotor No.: R15A, manufactured by Hitachi Koki Co., Ltd.), under the conditions of 25°C, 5,000 r/min, and 3 minutes. the centrifuge tube in which the defoamed surfactant composition was placed was allowed to stand still sideways for a few minutes, then immediately the centrifuge tube was erected, and the occurrence or nonoccurrence of the flow of the surfactant composition in the centrifuge tube at that time was visually observed, and the fluidity was evaluated according to the following criteria.
A: The flow of the surfactant composition is discerned (fluidity is discerned).
B: The flow of the surfactant composition is not discerned (no fluidity is discerned).

### <<Measurement of Viscosity>>

After the evaluation of the foregoing fluidity, the viscosity (Pa·s) of each of the surfactant compositions each placed in a centrifuge tube was measured by using a helical type viscometer "VHS-1" (rotor No.: T=D, manufactured by Toki Sangyo Co., Ltd.), under the conditions of 25°C and 5.0 r/min.

### <<Evaluation of Storage Stability>>

According to an acceleration test method using centrifugation, by using each of the surfactant compositions placed in the centrifuge tubes, the evaluation of the storage stabilities of each of the surfactant compositions in the temperature regions at 40°C, 25°C, and 15°C was performed.

For the samples stored at 40°C, used were the foregoing centrifuge tubes subjected to centrifugation treatment at 5,000 r/min, by using a centrifuge "MODEL SL-IVDH" (rotor No.: BT-1, manufactured by Sakuma Seisakusho Co., Ltd.), with a centrifuge chamber temperature set at 40°C. In this evaluation, when the sample thus treated was stored for 10 minutes, 30 minutes and 60 minutes, it was considered as the storage period of 1 month, 3 months and 6 months, respectively as the converted centrifuge time. The stored samples at the elapsed times of 10 minutes (corresponding to 1 month), 30 minutes (corresponding to 3 months) and 60 minutes (corresponding to 6 months) were visually evaluated according to the following criteria.

For the samples stored at 25°C, used were the foregoing centrifuge tubes subjected to centrifugation treatment at 5,000 r/min, by using a centrifuge "Hitachi high speed refrigerated centrifuge, CR21GIII" (rotor No.: R15A, manufactured by Hitachi Koki Co., Ltd.), with a centrifuge chamber temperature set at 25°C. In this evaluation, when the sample thus treated was stored for 12 minutes, 36 minutes and 72 minutes, it was considered as the storage period of 1 month, 3 months and 6 months, respectively as the converted centrifuge time. The stored samples at the elapsed times of 12 minutes (corresponding to 1 month), 36 minutes (corresponding to 3 months) and 72 minutes (corresponding to 6 months) were visually evaluated according to the following criteria.

Further, for the samples stored at 15°C, the samples treated in the same manner as in the samples stored at 25°C except that the chamber temperature was set at 15°C were used, and were evaluated in the same manner as in the samples stored at 25°C.
A: The sample appears uniform after the centrifugation corresponding to 6-month storage, and is recognized free from phase separation or deposition.
B: The sample appears uniform after the centrifugation corresponding to the 3-month storage, but is recognized to have phase separation or deposition after the centrifugation corresponding to 6-month storage.
C: The sample appears uniform after the centrifugation corresponding to 1-month storage, but is recognized to have phase separation or deposition after the centrifugation corresponding to 3-month storage.
D: The sample is recognized to have phase separation or deposition after the centrifugation corresponding to 1-month storage.

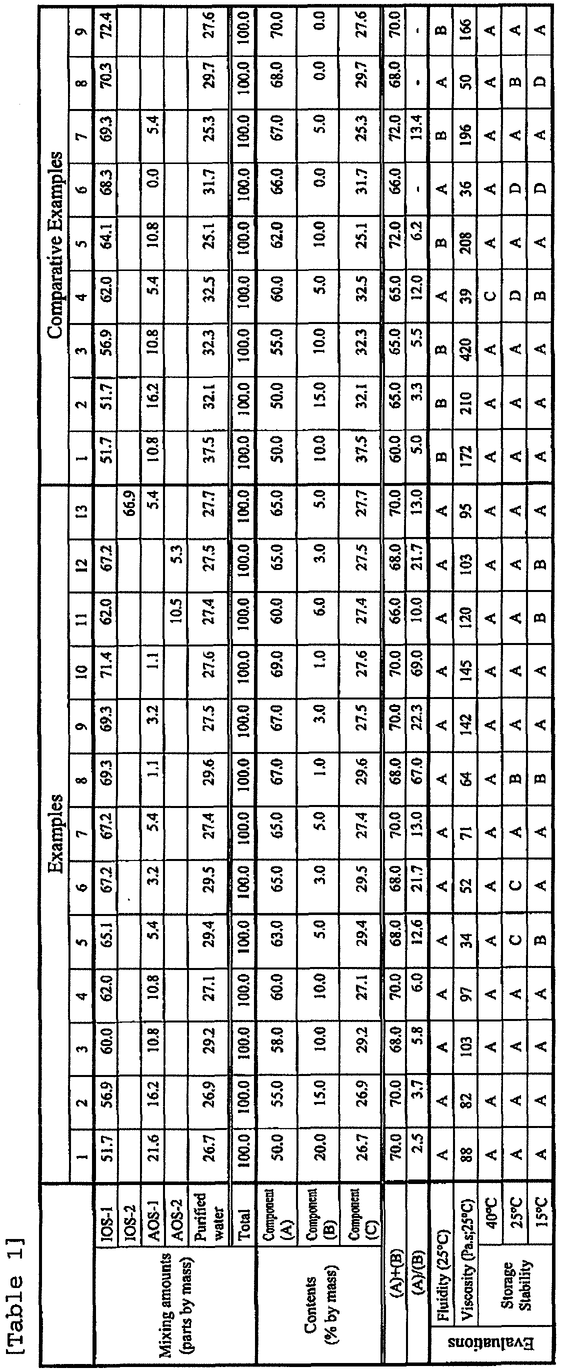

### [Formulation Example 1 (Hair shampoo)]

A hair shampoo having the following composition was prepared according to a conventional method by using the surfactant composition obtained in Example 7.

Specifically, purified water was placed in a beaker, cationized guar gum was added to the water and dispersed uniformly, then the resulting dispersion liquid was heated to 80°C; to the heated dispersion liquid, the surfactant composition obtained in Example 7, cocamidopropyl betaine, cocamide monoethanolamine, and sodium benzoate were added, and mixed uniformly. Subsequently, the resulting mixture was cooled to 40°C, and then ethylene glycol distearate was dispersed uniformly in the mixture, and a fragrance was added to the mixture. The moisture evaporated during mixing was regulated, and thus a hair shampoo having the following composition was obtained.

| (Components) | (% by mass) |
|---|---|
| The surfactant composition obtained in Example 7 | 20.0 |
| Cocamidopropyl betaine | 1.4 |
| Cocamide monoethanol amide | 0.6 |
| Cationized guar gum*¹ | 0.3 |
| Ethylene glycol distearate*² | 5.0 |
| Sodium benzoate | 0.3 |
| Fragrance | 0.4 |
| Purified water | 72.0 |
| Total | 100.0 |

| | |
|---|---|
| *1: "Jaguar C13S", manufactured by Sansho Co., Ltd. *2: "Pearl Concentrate FC-1" (content of ethylene glycol distearate: 20%) manufactured by Kao Corporation | |

The resulting hair shampoo was excellent in foamability, detergency, and was found to have an excellent feeling of use.

### [Industrial Applicability]

The surfactant composition of the present invention can be suitably used as a base material for detergents in a wide field of detergents such as a fabric detergent, a dish detergent, a hair cleaning detergent, a body cleaning detergent, a precision component detergent, and a hard surface detergent.

## Claims

1. A surfactant composition comprising the following components (A), (B), and (C):
(A) 45.0% by mass or more and 70.0% by mass or less of an internal olefin sulfonate having 16 or more and 18 or less carbon atoms,
(B) 0.5% by mass or more and 25.0% by mass or less of an alpha-olefin sulfonate having 12 or more and 14 or less carbon atoms, and
(C) water,
wherein the total content of the component (A) and the component (B) is 66.0% by mass or more and 71.0% by mass or less.

2. The surfactant composition according to claim 1, wherein the mass ratio of the content of the component (A) to the content of the component (B), ((A)/(B)), is 1.8 or more and 140 or less.

3. The surfactant composition according to claim 1 or 2, wherein the viscosity at 25°C is 5 Pa·s or more and 155 Pa·s or less.

4. The surfactant composition according to any one of claims 1 to 3, wherein the mass ratio of the content of the internal olefin sulfonate having 16 carbon atoms to the content of the internal olefin sulfonate having 18 carbon atoms, (internal olefin sulfonate having 16 carbon atoms/internal olefin sulfonate having 18 carbon atoms), in the component (A) is from 100/0 to 40/60.

5. The surfactant composition according to any one of claims 1 to 4, wherein the content of the internal olefin sulfonate having the sulfonate group at the position 2 in the component (A) is 30.0% by mass or less.

6. The surfactant composition according to any one of claims 1 to 5, wherein the mass ratio of the content of the hydroxy form of the internal olefin sulfonate to the content of the olefin form of the internal olefin sulfonate, (hydroxy form/olefin form), in the component (A) is from 50/50 to 100/0.

7. The surfactant composition according to any one of claims 1 to 6, wherein the content of the olefin sulfonate having the sulfonate group at the position 3 or a more inner position of the olefin chain or the alkane chain in the component (A) is 60.0% by mass or more.

8. The surfactant composition according to any one of claims 1 to 7, wherein the content of the olefin sulfonate having the sulfonate group at the position 1 of the olefin chain or the alkane chain in the component (A) is 3.0% by mass or less.

9. The surfactant composition according to any one of claims 1 to 8, wherein the total content of the component (A) and the component (B) is 69.0% by mass or more and 71. 0% by mass or less.

10. The surfactant composition according to any one of claims 1 to 9, wherein the content of the component (A) is 61.0% by mass or more and 69.0% by mass or less.

11. The surfactant composition according to any one of claims 1 to 10, wherein the content of the component (B) is 1.0% by mass or more and 6.0% by mass or less.

12. The surfactant composition according to any one of claims 1 to 11, wherein the mass ratio of the content of the component (A) to the content of the component (B), ((A)/(B)), is 2.5 or more and 75.0 or less.

13. Use of the surfactant composition according to any one of claims 1 to 12 as a base material for a detergent.

14. The use according to claim 13, wherein the base material for a detergent is a base material for a fabric detergent, a base material for a dish detergent, a base material for a hair cleaning detergent, a base material for a body cleaning detergent, a base material for a precision component detergent, or a base material for a hard surface detergent.

15. The use of the surfactant composition according to any one of claims 1 to 12 for production of a detergent.

## Patentansprüche

1. Tensidzusammensetzung, umfassend die folgenden Komponenten (A), (B) und (C):
(A) 45,0 Masse-% oder mehr und 70,0 Masse-% oder weniger eines innenständigen Olefinsulfonats mit 16 oder mehr und 18 oder weniger Kohlenstoffatomen,
(B) 0,5 Masse-% oder mehr und 25,0 Masse-% eines alpha-Olefinsulfonats mit 12 oder mehr und 14 oder weniger Kohlenstoffatomen und
(C) Wasser,
wobei der Gesamtgehalt der Komponente (A) und der Komponente (B) 66,0 Masse-% oder mehr und 71,0 Masse-% oder weniger beträgt.

2. Tensidzusammensetzung gemäß Anspruch 1, wobei das Masseverhältnis des Gehalts der Komponente (A) zu dem Gehalt der Komponente (B), ((A)/(B)), 1,8 oder mehr und 140 oder weniger beträgt.

3. Tensidzusammensetzung gemäß Anspruch 1 oder 2, wobei die Viskosität bei 25°C 5 Pa·s oder mehr und 155 Pa·s oder weniger beträgt.

4. Tensidzusammensetzung gemäß irgendeinem der Ansprüche 1 bis 3, wobei das Masseverhältnis des Gehalts des innenständigen Olefinsulfonats mit 16 Kohlenstoffatomen zu dem Gehalt des innenständigen Olefinsulfonats mit 18 Kohlenstoffatomen, (innenständiges Olefinsulfonat mit 16 Kohlenstoffatomen/innenständiges Olefinsulfonat mit 18 Kohlenstoffatomen), in der Komponente (A) 100/0 bis 40/60 beträgt.

5. Tensidzusammensetzung gemäß irgendeinem der Ansprüche 1 bis 4, wobei der Gehalt des innenständigen Olefinsulfonats mit der Sulfonatgruppe an der Position 2 in der Komponente (A) 30,0 Masse-% oder weniger beträgt.

6. Tensidzusammensetzung gemäß irgendeinem der Ansprüche 1 bis 5, wobei das Masseverhältnis des Gehalts der Hydroxyform des innenständigen Olefinsulfonats zu dem Gehalt der Olefinform des innenständigen Olefinsulfonats (Hydroxyfrom/Olefinform), in der Komponente (A) 50/50 bis 100/0 beträgt.

7. Tensidzusammensetzung gemäß irgendeinem der Ansprüche 1 bis 6, wobei der Gehalt des Olefinsulfonats mit der Sulfonatgruppe an der Position 3 oder einer weiter innenständigeren Position der Olefinkette oder der Alkankette in der Komponente (A) 60,0 Masse-% oder mehr beträgt.

8. Tensidzusammensetzung gemäß irgendeinem der Ansprüche 1 bis 7, wobei der Gehalt des Olefinsulfonats mit der Sulfonatgruppe an der Position 1 der Olefinkette oder der Alkankette in der Komponente (A) 3,0 Masse-% oder weniger beträgt.

9. Tensidzusammensetzung gemäß irgendeinem der Ansprüche 1 bis 8, wobei der Gesamtgehalt der Komponente (A) und der Komponente (B) 69,0 Masse-% oder mehr und 71,0 Masse-% oder weniger beträgt.

10. Tensidzusammensetzung gemäß irgendeinem der Ansprüche 1 bis 9, wobei der Gehalt der Komponente (A) 61,0 Masse-% oder mehr und 69,0 Masse-% oder weniger beträgt.

11. Tensidzusammensetzung gemäß irgendeinem der Ansprüche 1 bis 10, wobei der Gehalt der Komponente (B) 1,0 Masse-% oder mehr und 6,0 Masse-% oder weniger beträgt.

12. Tensidzusammensetzung gemäß irgendeinem der Ansprüche 1 bis 11, wobei das Masseverhältnis des Gehalts der Komponente (A) zu dem Gehalt der Komponente (B), ((A)/(B)), 2,5 oder mehr und 75,0 oder weniger beträgt.

13. Verwendung der Tensidzusammensetzung gemäß irgendeinem der Ansprüche 1 bis 12 als Basismaterial für ein Reinigungsmittel.

14. Verwendung gemäß Anspruch 13, wobei das Basismaterial für ein Reinigungsmittel ein Basismaterial für ein Waschmittel, ein Basismaterial für ein Geschirrspülmittel, ein Basismaterial für ein Haarreinigungsmittel, ein Basismaterial für ein Körperreinigungsmittel, ein Basismaterial für ein Präzisionskomponenten-Reinigungsmittel oder ein Basismaterial für ein Reinigungsmittel für harte Oberflächen ist.

15. Verwendung der Tensidzusammensetzung gemäß irgendeinem der Ansprüche 1 bis 12 zur Herstellung eines Reinigungsmittels.

## Revendications

1. Composition de tensioactif comprenant les composants (A), (B) et (C) suivants :
(A) 45,0 % en masse ou plus et 70,0 % en masse ou moins d'un sulfonate d'oléfine interne ayant un nombre d'atomes de carbone supérieur ou égal à 16 et inférieur ou égal à 18,
(B) 0,5 % en masse ou plus et 25,0 % en masse ou moins d'un sulfonate d'α-oléfine ayant un nombre d'atomes de carbone supérieur ou égal à 12 et inférieur ou égal à 14, et
(C) de l'eau,
dans laquelle la teneur totale en le composant (A) et en le composant (B) est supérieure ou égale à 66,0 % en masse et inférieure ou égale à 71,0 % en masse.

2. Composition de tensioactif selon la revendication 1, dans laquelle le rapport en masse de la teneur en composant (A) sur la teneur en composant (B), ((A)/B)), est supérieur ou égal à 1,8 et inférieur ou égal à 140.

3. Composition de tensioactif selon la revendication 1 ou 2, dans laquelle la viscosité à 25 °C est supérieure ou égale à 5 Pa·s et inférieure ou égale à 155 Pa·s.

4. Composition de tensioactif selon l'une quelconque des revendications 1 à 3, dans laquelle le rapport en masse de la teneur en sulfonate d'oléfine interne ayant 16 atomes de carbone sur la teneur en sulfonate d'oléfine interne ayant 18 atomes de carbone, (sulfonate d'oléfine interne ayant 16 atomes de carbone/sulfonate d'oléfine interne ayant 18 atomes de carbone), dans le composant (A) est de 100/0 à 40/60.

5. Composition de tensioactif selon l'une quelconque des revendications 1 à 4, dans laquelle la teneur en le sulfonate d'oléfine interne ayant le groupe sulfonate en position 2 dans le composant (A) est inférieure ou égale à 30,0 % en masse.

6. Composition de tensioactif selon l'une quelconque des revendications 1 à 5, dans laquelle le rapport en masse de la teneur en la forme hydroxy du sulfonate d'oléfine interne sur la teneur en la forme oléfinique du sulfonate d'oléfine interne (forme hydroxy/forme oléfinique), dans le composant (A) est de 50/50 à 100/0.

7. Composition de tensioactif selon l'une quelconque des revendications 1 à 6, dans laquelle la teneur en le sulfonate d'oléfine ayant le groupe sulfonate en position 3 ou une position plus interne de la chaîne oléfinique ou de la chaîne alcane dans le composant (A) est supérieure ou égale à 60,0 % en masse.

8. Composition de tensioactif selon l'une quelconque des revendications 1 à 7, dans laquelle la teneur en sulfonate d'oléfine ayant le groupe sulfonate en position 1 de la chaîne oléfinique ou de la chaîne alcane dans le composant (A) est inférieure ou égale à 3,0 % en masse.

9. Composition de tensioactif selon l'une quelconque des revendications 1 à 8, dans laquelle la teneur totale composant (A) et en composant (B) est supérieure ou égale à 69,0 % en masse et inférieure ou égale à 71,0 % en masse.

10. Composition de tensioactif selon l'une quelconque des revendications 1 à 9, dans laquelle la teneur en le composant (A) est supérieure ou égale à 61,0 % en masse et inférieure ou égale à 69,0 % en masse.

11. Composition de tensioactif selon l'une quelconque des revendications 1 à 10, dans laquelle la teneur en composant (B) est supérieure ou égale à 1,0 % en masse et inférieure ou égale à 6,0 % en masse.

12. Composition de tensioactif selon l'une quelconque des revendications 1 à 11, dans laquelle le rapport en masse de la teneur en composant (A) sur la teneur en composant (B), ((A)/(B)), est supérieur ou égal à 2,5 et inférieur ou égal à 75,0.

13. Utilisation de la composition de tensioactif selon l'une quelconque des revendications 1 à 12 en tant que matériau de base pour un détergent.

14. Utilisation selon la revendication 13, dans laquelle le matériau de base pour un détergent est un matériau de base pour un détergent pour textile, un matériau de base pour un détergent pour vaisselle, un matériau de base pour un détergent pour les cheveux, un matériau de base pour un détergent pour le corps, un matériau de base pour un détergent pour un composant de précision, ou un matériau de base pour un détergent pour surfaces dures.

15. Utilisation de la composition de tensioactif selon l'une quelconque des revendications 1 à 12 pour la production d'un détergent.
